# EUROPÄISCHE PATENTSCHRIFT

(11) **EP 3 774 807 B1**
(45) Veröffentlichungstag und Bekanntmachung des Hinweises auf die Patenterteilung: **01.09.2021**
(21) Anmeldenummer: 18772759.9
(22) Anmeldetag: 06.09.2018
(51) Int. Cl.: C07D 487/04

(54) **VERFAHREN ZUR HERSTELLUNG BICYCLISCHER GUANIDINE**
PROCESS FOR PREPARING BICYCLIC GUANIDINES
PROCÉDÉ DE PRODUCTION DE GUANIDINE BICYCLIQUE

(43) Veröffentlichungstag der Anmeldung: 17.02.2021
(73) Patentinhaber: Wacker Chemie AG, 81737 München (DE)
(72) Erfinder: FRITZ-LANGHALS, Elke, 85521 Ottobrunn (DE); SCHEIM, Uwe, 01640 Coswig (DE)
(74) Vertreter: Budczinski, Angelika
(86) Internationale Anmeldenummer: PCT/EP2018/074069
(87) Internationale Veröffentlichungsnummer: WO 2020/048604

(56) Entgegenhaltungen:
- US-A1- 2009 281 314
- US-A1- 2012 220 770
- US-A1- 2013 289 272

## Beschreibung

Die Erfindung betrifft ein Verfahren zur Herstellung bicyclischer Guanidine sowie bicyclische Guanidine enthaltende Formulierungen und deren Verwendung als Katalysator.

Bei zahlreichen industriell durchgeführten chemischen Prozessen werden Basen als Katalysatoren eingesetzt. Vor allem im Bereich der Polymerchemie werden organische Basen bevorzugt, da sie in unpolarem Milieu wesentlich besser löslich sind als anorganische Basen wie z.B. Natrium- oder Kaliumhydroxid, welche außerdem unerwünschte Nebenreaktionen auslösen können.

Für eine möglichst große katalytische Wirkung ist eine große Basenstärke erwünscht. Daher sind bicyclische Guanidine wie z.B. 1,5,7-Triazabicyclo[4.4.0]-dec-5-en (TBD) prinzipiell gegenüber den gängigeren, jedoch deutlich schwächer basischen Verbindungen Tetramethylguanidin, DBU (Diazabicycloundecen) oder DBN (Diazabicyclodecen) bevorzugt.

TBD ist beispielsweise ein hochwirksamer Katalysator für die Ringöffnungspolymerisation von Lactonen und cyclischen Siloxanen, außerdem bei der Herstellung von Polyurethanen. Gemäß DE 10 2015216 598 A1 kann das Endcapping von Hydroxypolysiloxanen mit TBD effizient katalysiert werden.

Dem technischen Einsatz von TBD und weiteren bicyclischen Guanidinen stand bisher jedoch deren mangelnde technische Zugänglichkeit im Wege. TBD kann beispielsweise durch Umsetzung von Dipropylentriamin mit den C1-Bausteinen Schwefelkohlenstoff, Carbodiimiden und Guanidinen hergestellt werden. Bei der in US 4,797,487 beschriebenen Schwefelkohlenstoff-Route entsteht jedoch toxisches Schwefelwasserstoffgas, die Minimierung des Risikos dessen unbeabsichtigter Freisetzung erfordert einen hohen technischen Aufwand. Ein weiteres Sicherheitsrisiko stellt der niedrige Flammpunkt von Schwefelkohlenstoff dar.

Die in den US 20130289272 und US 2013163130 beschriebenen Routen ausgehend von Carbodiimiden sind aufgrund des hohen Preises von Diimiden unwirtschaftlich. Zudem können Verunreinigungen nur sehr schwer abgetrennt werden.

Bei der beispielsweise in der US 2012/0259112 beschriebenen Synthese bicyclischer Guanidine durch Einsatz acyclischer Guanidinsalze als C1-Bausteine entstehen große Mengen Ammoniak, welche aufgefangen und entsorgt werden müssen, was das Verfahren ebenfalls erheblich verteuert. Zudem fällt TBD als Salz an, welches in einem zusätzlichen Schritt in die freie Base überführt werden muss.

In den Veröffentlichungen US 2009/0281313 und US 2009/0281314 wird die Herstellung von TBD aus Dipropylentriamin mit Dimethylcarbonat beschrieben. Gegenüber den oben genannten Routen besitzt diese Route den Vorteil, dass kostengünstige großtechnisch verfügbare Edukte eingesetzt werden können und keine toxischen Gase bei der Umsetzung entstehen. Zunächst bildet sich bei der Umsetzung unter zweifacher Methanolabspaltung ein cyclischer Harnstoff und bei Temperaturen > 200°C und Reaktionszeiten von bis zu 50 Stunden die Bildung von TBD, wobei als Reaktionsmedium bevorzugt hochsiedende Ether eingesetzt werden, beispielsweise Glycolether wie Triethylenglycoldimethylether und Diethylenglycolmonobutylether oder auch Glycoletheracetale wie Butylcarbitolformal. Diese ermöglichen eine druckfreie Ausführung der Reaktion auch bei den hohen Temperaturen > 200°C. Das Produkt TBD wird nach der Umsetzung als Feststoff isoliert, indem entweder das Lösemittel entfernt wird und sich das Produkt TBD im Rückstand befindet, oder das Produkt durch Zusatz eines Fällungsmittels, z.B. eines Kohlenwasserstoffs, auskristallisiert wird. In weiteren Ausführungsformen wird die Reaktionsmischung in dem hochsiedenden Ether zusätzlich mit einem Reagens zur Bindung des gebildeten Wassers versetzt, als Beispiele für dieses werden Disilazane und Tetraethoxysilan (TEOS) genannt. Bei der Verwendung von TEOS bildet sich jedoch, wie in der US 2009/0281314 Abschnitt [0028] offenbart, unter den dort angegebenen Bedingungen unlösliches Silica, welches nur über Filtration der verdünnten Produktlösung abgetrennt werden kann, was den Prozess weiter verteuert. Hinzu kommt, dass die Verwendung von Glycolethern als Lösemittel problematisch ist, da diese in Kontakt mit Luft zur Bildung von thermisch labilen Etherperoxiden neigen und außerdem toxisch sind. Diese Lösemittel müssen daher vor der Anwendung möglichst vollständig entfernt werden, was mehrfaches Umkristallisieren erfordert. Die Herstellung des Produkts wird damit erheblich verteuert und dessen Einsatz letztlich unwirtschaftlich.

Insbesondere für Anwendungen in der Polymerchemie ist das kristalline bicyclische Guanidin nur wenig geeignet, da es sich nur sehr langsam in Polymeren, insbesondere in Siloxanen löst. Durch den langwierigen Prozess des Lösens vergrößert sich der technische Aufwand beträchtlich, was letztlich zur Unwirtschaftlichkeit des Verfahrens führen kann. Es besteht auch stets die Gefahr, dass unlösliche Bestandteile des Katalysators im Reaktionsgemisch verbleiben, dies hat dann einen Mehrverbrauch zur Folge, was wiederum die Wirtschaftlichkeit herabsetzt.

Es bestand daher die Aufgabe, ein Verfahren zur Herstellung von bicyclischen Guanidinen bereitzustellen, welches die o.g. Nachteile nicht aufweist und eine kostengünstige und technisch einfache Produktion ermöglicht. Eine weitere Aufgabe ist, eine auf einfachem Weg kostengünstig herstellbare flüssige Formulierung von bicyclischem Guanidin bereitzustellen, welche direkt für katalytische Zwecke eingesetzt werden kann.

Gegenstand der Erfindung ist ein Verfahren zur Herstellung bicyclischer Guanidine durch Umsetzung von (A) Dialkylentriamin mit (B) Dialkylcarbonat

in Gegenwart von (C) Silan der Formel

Si (OR^{x})ₒR^{y}₍₄₋ₒ₎ (IV)

und/oder dessen Teilhydrolysate,
wobei
R^{x} gleich oder verschieden sein können und einwertige, gegebenenfalls substituierte Kohlenwasserstoffreste mit 2 bis 10 Kohlenstoffatomen darstellen,
R^{y} gleich oder verschieden sein können und einwertige, gegebenenfalls substituierte Kohlenwasserstoffreste mit 1 bis 30 Kohlenstoffatomen, bei denen einzelne nicht an Silicium gebundene CH₂-Gruppierungen durch Sauerstoff ersetzt oder durch Silylgruppen substituiert sein können, darstellen und
o 1, 2, 3 oder 4, bevorzugt 2, 3 oder 4, besonders bevorzugt 3, ist,
mit der Maßgabe, dass bei Silanen der Formel (IV) mit o=4 mindestens zwei Reste R^{x} die Bedeutung von einwertigem, gegebenenfalls substituiertem Kohlenwasserstoffrest mit 3 bis 10 Kohlenstoffatomen haben.

Vorzugsweise handelt es sich bei dem erfindungsgemäß eingesetzten Dialkylentriamin (A) um solche der allgemeinen Formel

H₂N- (CR^{a}₂)ₘ-CR^{a}₂-NH-CR^{a}₂- (CR^{a}₂)ₙ-NH₂ (II),

wobei
m und n unabhängig voneinander 1, 2, 3 oder 4, bevorzugt 1, 2 oder 3, besonders bevorzugt 1 oder 2, sind und R^{a} gleich oder verschieden sein kann und Wasserstoffatom oder einwertige Kohlenwasserstoffreste darstellt, wobei einzelne Methylengruppen durch Sauerstoff oder durch Gruppierungen -NH- oder -NR^{d}- ersetzt sein können, wobei R^{d} einwertige, gegebenenfalls substituierte Kohlenwasserstoffreste mit 2 bis 10 Kohlenstoffatomen bedeutet.

Beispiele für einwertige R^{a} sind Alkylreste, wie der Methyl-, Ethyl-, n-Propyl-, iso-Propyl-, 1-n-Butyl-, 2-n-Butyl-, isoButyl-, tert.-Butyl-, n-Pentyl-, iso-Pentyl-, neo-Pentyl-, tert.-Pentylrest; Hexylreste, wie der n-Hexylrest, der 2-Methylpentylrest; Heptylreste, wie der n-Heptylrest; Octylreste, wie der n-Octylrest und iso-Octylreste, wie der 2,2,4-Trimethylpentylrest; Nonylreste, wie der n-Nonylrest; Decylreste, wie der n-Decylrest; Undecylreste wie der n-Undecylrest, Dodecylreste, wie der n-Dodecylrest; Octadecylreste, wie der n-Octadecylrest; Cycloalkylreste, wie der Cyclopentyl-, Cyclohexyl-, Cycloheptylrest und Methylcyclohexylreste; Alkenylreste, wie der Vinyl-, 1-Propenyl- und der 2-Propenylrest; Arylreste, wie der Phenyl-, Naphthyl-, Anthryl- und Phenanthrylrest; Alkarylreste, wie o-, m-, p-Tolylreste; Xylylreste und Ethylphenylreste; und Aralkylreste, wie der Benzylrest, der α- und der β-Phenylethylrest, -OH, -OCH₃, -OC2H₅, -CH₂-O-CH₃, -NH₂, -CH₂-NH₂ oder -CH₂-N(CH₃)₂.

Bevorzugt handelt es sich bei den Resten R^{a} um Wasserstoffatom oder Kohlenwasserstoffreste mit 1 bis 10 Kohlenstoffatomen, wobei einzelne Methylengruppen durch Sauerstoff oder durch Gruppierungen -NH- oder -NR^{d}- ersetzt sein können, wobei R^{d} die oben dafür angegebene Bedeutung hat, besonders bevorzugt um Wasserstoffatom oder aliphatische lineare oder verzweigte Kohlenwasserstoffreste mit 1 bis 6 Kohlenstoffatomen, insbesondere um Wasserstoffatom.

Bevorzugt hat im Triamin (A) der Formel (II) m die gleiche Bedeutung wie n, wobei m=n= 1 oder 2 besonders bevorzugt ist.

Bevorzugt handelt es sich bei dem erfindungsgemäß eingesetzten Triamin (A) um Bis-(3-aminopropyl)amin, Bis-(2-aminoethyl)amin, 1-Amino-3-[(3-amino-2-hydroxypropyl)amino]propan-2-ol oder N-(2-Aminoethyl)-N-(3-aminopropyl)amin, wobei Bis-(3-aminopropyl)amin besonders bevorzugt ist.

Vorzugsweise handelt es sich bei dem erfindungsgemäß eingesetzten Dialkylcarbonat (B) um solche der allgemeinen Formel

R^{b}O-CO-OR^{b} (III),

wobei
R^{b} gleich oder verschieden sein kann und ein- oder zweiwertige, aliphatisch gesättigte Kohlenwasserstoffreste darstellt.

Falls R^{b} die Bedeutung von zweiwertigen aliphatisch gesättigten Kohlenwasserstoffresten hat, sind diese bevorzugt miteinander verbunden und bilden über die beiden Sauerstoffatome einen Ring.

Beispiele für Reste R^{b} sind die für Rest R^{a} angegebenen Beispiele für einwertige aliphatisch gesättigte Kohlenwasserstoffreste sowie zweiwertige aliphatisch gesättigte Kohlenwasserstoffreste, wie -CH₂-(CH₂)ₚ-CH₂- mit p bevorzugt gleich 0, 1, 2, 3 oder 4, besonders bevorzugt 0 oder 1, wobei die zweiwertigen Reste R^{b} bevorzugt miteinander verbunden sind und über die beiden Sauerstoffatome einen Ring bilden.

Bevorzugt handelt es sich bei Rest R^{b} um ein- oder zweiwertige aliphatisch gesättigte Kohlenwasserstoffreste mit 1 bis 10 Kohlenstoffatomen, besonders bevorzugt um ein- oder zweiwertige aliphatisch gesättigte lineare oder verzweigte Kohlenwasserstoffreste mit 1 bis 6 Kohlenstoffatomen, insbesondere um den Methyl-, Ethyl-, Propyl-, Ethylen- oder Propylenrest, ganz besonders bevorzugt um den Methyl- oder Ethylrest.

Bevorzugt handelt es sich bei dem erfindungsgemäß eingesetzten Carbonat (B) um Dimethylcarbonat, Diethylcarbonat, Di-n-Propylcarbonat, Ethylencarbonat oder Propylencarbonat, wobei Dimethylcarbonat oder Diethylcarbonat besonders bevorzugt sind.

Bei dem erfindungsgemäßen Verfahren wird Dialkylcarbonat (B) in molaren Mengen von bevorzugt 0,5 bis 2, 0 Mol, besonders bevorzugt 0,8 bis 1,5 Mol, jeweils bezogen auf 1 Mol des eingesetzten Dialkylentriamins (A), eingesetzt.

Bevorzugt handelt es sich bei Rest R^{x} um lineare, verzweigte oder cyclische gesättigte oder ungesättigte Kohlenwasserstoffreste mit 2 bis 6 Kohlenstoffatomen, besonders bevorzugt um aliphatisch gesättigte lineare oder verzweigte Kohlenwasserstoffreste mit 2 bis 6 Kohlenstoffatomen, insbesondere um den Ethyl-, n-Propyl-, i-Propyl, n-Butyl oder 2-Butylrest, ganz besonders bevorzugt um den Ethylrest.

Beispiele für Reste R^{y} sind die für Rest R^{a} angegebenen Beispiele.

Bevorzugt handelt es sich bei den Resten R^{y} um lineare, verzweigte oder cyclische gesättigte oder ungesättigte Kohlenwasserstoffreste mit 1 bis 20 Kohlenstoffatomen, besonders bevorzugt um aliphatische, lineare oder verzweigte Kohlenwasserstoffreste mit 1 bis 12 Kohlenstoffatomen.

Beispiele für erfindungsgemäß eingesetzte Silane (C) sind *i-*Octyl-Si(OEt)₃, Methyltriethoxysilan, Ethyltriethoxysilan, Vinyltriethoxysilan, Propyltriethoxysilan, Butyltriethoxysilan, Cyclohexyltriethoxysilan, 2-Methylpropyltriethoxysilan, Pentyltriethoxysilan, Methyltris(1-methylethoxy)silan, n-Octyltriethoxysilan, Phenyltriethoxysilan, Benzyltriethoxysilan, Si(O-2-Butyl)₄, Si(OEt) (O-2-Butyl)₃, Si(OEt)₂(O-2-Butyl)₂, Si(OiProp)₄, Si(OEt) (OiProp)₃, Si(OEt)₂(OiProp)₂ und (EtO)₃Si-CH₂-CH₂-Si(OEt)₃, (EtO)₃Si-CH₂-CH₂-SiMe₃, (EtO)₂MeSi-CH₂-CH₂-SiMe(OEt)₂ und deren Teilhydrolysate, wobei Et gleich Ethylrest und Prop gleich Propylrest bedeutet.

Bevorzugt handelt es sind bei den erfindungsgemäß eingesetzten Silanen (C) um solche mit o=3 und R^{x} gleich einwertigem, aliphatisch gesättigtem, linearem oder verzweigtem Alkylrest mit 2 bis 4 Kohlenstoffatomen und R^{y} gleich einwertigem, aliphatischem, linearem oder verzweigtem Kohlenwasserstoffrest mit 6 bis 10 Kohlenstoffatomen oder um solche mit o=4 und mindestens zwei Resten R^{x} gleich einwertigem, aliphatisch gesättigtem, linearem oder verzweigtem Kohlenwasserstoffrest mit 3 bis 8 Kohlenstoffatomen und/oder jeweils deren Teilhydrolysate.

Besonders bevorzugt handelt es sind bei den erfindungsgemäß eingesetzten Silanen (C) um *i*-Octyl-Si(OEt)₃, n-Heptyl-Si(OEt)₃, n-Decyl-Si(OEt)₃, Phenyl-Si(OEt)₃, Si(O-2-Butyl)₄, Si(OEt)(O-2-Butyl)₃, Si(OEt)₂(O-2-Butyl)₂, Si(OiProp)₄, Si(OEt)(OiProp)₃ oder Si(OEt)₂(OiProp)₂ und/oder deren Teilhydrolysate, insbesondere um *i*-Octyl-Si(OEt)₃ und/oder dessen Teilhydrolysate.

Falls es sich bei Komponente (C) um Teilhydrolysate der Silane der Formel (IV) handelt, sind solche mit 2 bis 5 Siliciumatomen bevorzugt.

Bei dem erfindungsgemäßen Verfahren wird Komponente (C) in Mengen von bevorzugt 30 bis 1000 Gewichtsteilen, besonders bevorzugt 100 bis 500 Gewichtsteilen, jeweils bezogen auf 100 Gewichtsteile Dialkylentriamin (A), eingesetzt.

Im erfindungsgemäßen Verfahren können zusätzlich zu den Komponenten (A), (B) und (C) weitere Stoffe eingesetzt werden, die jeweils unterschiedlich sind zu den Komponenten (A), (B) und (C), wie z.B. Basen (D) oder organische Lösungsmittel (E).

Beispiele für gegebenenfalls eingesetzte Basen (D) sind anorganische oder organische Basen.

Bevorzugt handelt es sich bei den gegebenenfalls eingesetzten Basen (D) um Stickstoffbasen, die unterschiedlich sind zu Komponente (A), besonders bevorzugt um TBD, DBU, DBN, Pyridin oder Dimethylaminpyridin, Guanidin, Tetramethylguanidin oder Tetraethylguanidin.

Falls im erfindungsgemäßen Verfahren Basen (D) eingesetzt werden, handelt es sich um Mengen von vorzugsweise 0,01 bis 20 Gewichtsteilen, besonders bevorzugt 0,1 bis 10 Gewichtsteilen, insbesondere 1 bis 5 Gewichtsteilen, jeweils bezogen auf 100 Gewichtsteile an eingesetztem Dialkylentriamin (A). Beim erfindungsgemäßen Verfahren wird bevorzugt keine Base (D) eingesetzt.

Vorzugsweise handelt es sich bei den gegebenenfalls eingesetzten Lösungsmitteln (E) um Alkohole, Phenole, Nitrile, Dialkyl- oder Diarylether oder Kohlenwasserstoffe, wobei Alkohole oder Kohlenwasserstoffe bevorzugt und Alkohole besonders bevorzugt sind.

Falls im erfindungsgemäßen Verfahren Lösungsmittel (E) eingesetzt werden, handelt es sich um Mengen von vorzugsweise 0,1 bis 200 Gewichtsteilen, besonders bevorzugt 1 bis 100 Gewichtsteilen, insbesondere 10 bis 50 Gewichtsteilen, jeweils bezogen auf 100 Gewichtsteile des eingesetzten Dialkylentriamins (A). Beim erfindungsgemäßen Verfahren wird bevorzugt kein Lösungsmittel (E) eingesetzt.

Falls erwünscht, können im erfindungsgemäßen Verfahren noch weitere Stoffe eingesetzt werden, wie z.B. die im Zusammenhang mit der erfindungsgemäßen Zubereitung weiter unten beschriebenen Füllstoffe (f), Farbmittel (g) und Polymere (h), was jedoch nicht bevorzugt ist.

Im erfindungsgemäßen Verfahren werden bevorzugt keine Glycolether eingesetzt.

Im erfindungsgemäßen Verfahren werden bevorzugt keine über die Komponenten (A) bis (E) sowie (f), (g) und (h) hinausgehenden weiteren Bestandteile, besonders bevorzugt keine über die Komponenten (A) bis (E) hinausgehenden weiteren Bestandteile eingesetzt.

Bei den im erfindungsgemäßen Verfahren eingesetzten Komponenten kann es sich jeweils um eine Art einer solchen Komponente wie auch um ein Gemisch aus mindestens zwei Arten einer jeweiligen Komponente handeln.

Bei dem erfindungsgemäßen Verfahren werden bevorzugt in einer ersten Stufe die Komponenten (A) und (B) sowie gegebenenfalls Komponenten (D), (E), (f), (g) und (h) vermischt und reagieren gelassen und dann in einer zweiten Stufe Komponente (C) sowie gegebenenfalls Komponenten (D), (E), (f), (g) und (h) zugegeben und auf eine Temperatur größer 200°C erhitzt.

In der ersten Stufe des erfindungsgemäßen Verfahrens wird Komponente (A) sowie gegebenenfalls Komponenten (D), (E), (f), (g) und (h) bevorzugt bei Temperaturen zwischen Raumtemperatur und 120°C vorgelegt und Komponente (B) zugegeben, wobei eine exotherme Reaktion stattfindet, wodurch die Temperatur des Reaktionsgemischs weiter ansteigt. Bevorzugt wird die Temperatur gegebenenfalls durch Kühlen oder Erwärmen auf 20 bis 120°C gehalten, wobei der entstehende Alkohol, vorzugsweise R^{b}-OH, abgetrennt, bevorzugt abdestilliert, wird. Der gebildete Alkohol kann auch im Anschluss an die Reaktion abgetrennt werden, bevorzugt durch Destillation. Bei dieser Umsetzung bildet sich durch Abspaltung von Alkohol ein cyclischer Harnstoff.

In der zweiten Stufe des erfindungsgemäßen Verfahrens wird zu der in der ersten Stufe erhaltenen Reaktionsmischung Komponente (C) sowie gegebenenfalls Komponenten (D), (E), (f), (g) und (h) zugegeben und auf eine Temperatur von bevorzugt 200°C bis 280°C, besonders bevorzugt von 210°C bis 260°C, insbesondere auf eine Temperatur von 220°C bis 250°C, aufgeheizt und reagieren gelassen, wobei Alkohol R^{x}-OH entsteht, der vorzugsweise entfernt wird. Das Entfernen des Alkohols erfolgt dabei vorzugsweise durch Destillation, besonders bei Drucken zwischen 0,1 mbar und 50 bar, besonders bevorzugt bei Drucken zwischen 1 mbar und 20 bar, ganz besonders bevorzugt bei Umgebungsdruck.

In einer weiteren Durchführungsform des erfindungsgemäßen Verfahrens werden alle Komponenten in beliebiger Reihenfolge miteinander vermischt und reagieren gelassen, wobei die Temperatur bevorzugt nach dem Abklingen der exothermen Reaktion im Zeitraum von bevorzugt 0,1 bis 10 Stunden, besonders bevorzugt 1 bis 5 Stunden, auf 220 bis 280°C erhöht wird und in diesem Temperaturbereich für bevorzugt 5 bis 30 Stunden, besonders bevorzugt 8 bis 20 Stunden, gehalten wird.

Das erfindungsgemäße Verfahren wird bevorzugt unter Schutzgas, wie z.B. Stickstoff, durchgeführt.

Das erfindungsgemäße Verfahren kann kontinuierlich, diskontinuierlich oder semikontinuierlich durchgeführt werden, wobei die diskontinuierliche Verfahrensweise bevorzugt ist.

Nach der erfindungsgemäßen Umsetzung wird eine blassgelbe Reaktionsmischung erhalten, aus der das bicyclische Guanidin bei Umgebungstemperatur nahezu quantitativ in kristalliner Form ausfällt, es kann daher auf einfache Weise, beispielsweise durch Filtration, abgetrennt werden. Es ist jedoch ebenso möglich, das bicyclische Guanidin durch fraktionierte Destillation, Sublimation oder Feststoffdestillation zu isolieren. Die Bildung von unlöslichen Komponenten wie z.B. Silica findet bei dem erfindungsgemäßen Verfahren bevorzugt nicht statt.

Bevorzugt werden nach dem erfindungsgemäßen Verfahren bicyclische Guanidine der Formel (I) 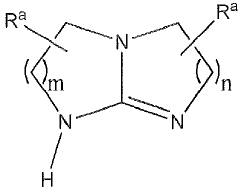 erhalten, wobei R^{a}, m und n die oben genannte Bedeutung haben.

Bei dem erfindungsgemäßen Verfahren können die abgetrennten Alkohole, bevorzugt R^{b}-OH und R^{x}-OH, wiederverwendet werden, z.B. bei der Herstellung von Alkoxysilanen oder bei der Herstellung der erfindungsgemäßen Zubereitung als Komponente (c). In diesem Fall entstehen durch das erfindungsgemäße Verfahren keinerlei Abfallprodukte, was einen besonderen Vorteil bei dem erfindungsgemäßen Verfahren darstellt.

In einer bevorzugten Ausführungsform wird die erhaltene Reaktionsmischung nach Reaktionsende mit Wasser oder ein- oder mehrwertigem Alkohol vermischt. Eine Aufarbeitung des Reaktionsgemisches kann dadurch vorteilhafterweise vollständig entfallen und die so erhaltene Zubereitung kann direkt eingesetzt werden.

Ein weiterer Gegenstand der Erfindung ist daher eine Zubereitung bestehend aus
(a) bicyclischem Guanidin,
(b) Silan der allgemeinen Formel (IV) und/oder dessen Teilhydrolysate,
(c) Verbindung R^{c}OH, wobei Rest R^{c} gleich Wasserstoffatom oder einwertige, gegebenenfalls substituierte Kohlenwasserstoffreste mit 1 bis 30 Kohlenstoffatomen, die mit Sauerstoff unterbrochen sein können, darstellt,
gegebenenfalls (d) Reaktionsnebenprodukte,
gegebenenfalls (e) organische, von an aliphatische Kohlenstoffatome gebundenen Hydroxylgruppen freie Lösungsmittel,
gegebenenfalls (f) Füllstoffe,
gegebenenfalls (g) Farbmittel und
gegebenenfalls (h) Polymere.

Zusätzlich zu den Komponenten (a), (b) und (c) sowie gegebenenfalls (e), (f) und (h) können die erfindungsgemäßen Zubereitungen Reaktionsnebenprodukte (d) aus der erfindungsgemäßen Umsetzung von (A) mit (B) enthalten, die vorzugsweise dann anwesend sind, wenn das nach dem erfindungsgemäßen Verfahren erhaltene Reaktionsgemisch ohne Aufarbeitung zur Herstellung der erfindungsgemäßen Zubereitungen verwendet wird, was bevorzugt ist.

Bei erfindungsgemäß eingesetzter Komponente (a) handelt es sich bevorzugt um Verbindungen der Formel (I), besonders bevorzugt um TBD.

Bei den erfindungsgemäßen Zubereitungen beträgt der Gehalt an bicyclischen Guanidinen (a) bevorzugt 2 bis 35 Gew.-%, besonders bevorzugt 5 bis 25 Gew.-%.

Bei erfindungsgemäß eingesetzter Komponente (b) handelt es sich bevorzugt um Silane der Formel (IV) im Gemisch mit deren durch Hydrolyse und Kondensation gebildeten Siloxanen.

Bei den erfindungsgemäßen Zubereitungen beträgt der Gehalt an Silanen und/oder Siloxanen (b) bevorzugt 20 bis 90 Gew.-%, besonders bevorzugt 30 bis 70 Gew.-%.

Beispiele für Rest R^{c} sind die für Rest R^{y} oben angegebenen Reste.

Bevorzugt handelt es sich bei Rest R^{c} um Wasserstoffatom oder aliphatisch gesättigte oder aliphatisch ungesättigte, lineare oder verzweigte oder cyclische, gegebenenfalls substituierte Kohlenwasserstoffreste mit 1 bis 12 Kohlenstoffatomen, die mit Sauerstoff unterbrochen sein können, besonders bevorzugt um aliphatisch gesättigte oder aliphatisch ungesättigte, lineare oder verzweigte, gegebenenfalls mit Hydroxylgruppen substituierte Kohlenwasserstoffreste mit 1 bis 6 Kohlenstoffatomen, die mit Sauerstoffatomen unterbrochen sein können, insbesondere um Wasserstoffatom, den Methyl-, Ethyl-, 2-Hydroxyethyl-, n-Propyl-, i-Propyl-, 2-Hydroxypropyl-, 2,3-Dihydroxypropyl-, n-Butyl- oder 2-Butyl-rest.

Bevorzugt handelt es sich bei Komponente (c) um Wasser, Methanol, Ethanol, n-Propanol, i-Propanol, Glycerin, Ethylenglycol oder Propylenglycol, wobei Ethanol besonders bevorzugt ist.

Bei den erfindungsgemäßen Zubereitungen beträgt der Gehalt an Komponente (c) bevorzugt 5 bis 50 Gew.-%, besonders bevorzugt 10 bis 30 Gew.-%.

Bevorzugt beträgt der Gewichtsanteil der Komponenten (a), (b), (c) und gegebenenfalls (d) in den erfindungsgemäßen Zusammensetzungen mindestens 80 Gew.-%, besonders bevorzugt mindestens 90 Gew.-%, insbesondere 100 Gew.-%.

Falls die erfindungsgemäßen Zubereitungen Reaktionsnebenprodukte (d) enthalten, handelt es sich um Mengen von vorzugsweise 1 bis 20 Gewichtsteilen, besonders bevorzugt 2 bis 10 Gewichtsteilen, jeweils bezogen auf 100 Gewichtsteile des Gesamtgewichts der Komponenten (a), (b) und (c).

Beispiele für gegebenenfalls eingesetzte Komponente (e) sind die oben für organische Lösungsmittel (E) angegebenen Beispiele, ausgenommen Alkohole.

Falls die erfindungsgemäßen Zubereitungen organische Lösungsmittel (e) enthalten, handelt es sich um Mengen von vorzugsweise 0,01 bis 100 Gewichtsteilen, besonders bevorzugt 0,1 bis 50 Gewichtsteilen, insbesondere 1 bis 10 Gewichtsteilen, jeweils bezogen auf 100 Gewichtsteile des Gesamtgewichts der Komponenten (a), (b) und (c). Die erfindungsgemäßen Zubereitungen enthalten bevorzugt kein organisches Lösungsmittel (e).

Beispiele für gegebenenfalls eingesetzte Füllstoffe (f) sind nicht verstärkende Füllstoffe, also Füllstoffe mit einer BET-Oberfläche von bis zu 50 m²/g, wie Quarz, Diatomeenerde, Calciumsilikat, Zeolithe, Siliciumnitrid, Siliciumcarbid, Bornitrid, Glaspulver; verstärkende Füllstoffe, also Füllstoffe mit einer BET-Oberfläche von mindestens 50 m²/g, wie pyrogen hergestellte Kieselsäure, gefällte Kieselsäure, und Silicium-Aluminium-Mischoxide großer BET-Oberfläche, wobei gefällte und pyrogene Kieselsäure bevorzugt und pyrogene Kieselsäure besonders bevorzugt ist.

Falls die erfindungsgemäßen Zubereitungen Füllstoffe (f) enthalten, handelt es sich um Mengen von vorzugsweise 0,1 bis 100 Gewichtsteilen, besonders bevorzugt 1 bis 50 Gewichtsteilen, insbesondere 5 bis 20 Gewichtsteilen, jeweils bezogen auf 100 Gewichtsteile des Gesamtgewichts der Komponenten (a), (b) und (c). Die erfindungsgemäßen Zubereitungen enthalten bevorzugt keinen Füllstoff (f).

Beispiele für gegebenenfalls eingesetzte Farbmittel (g) sind Farbstoffe wie Phthalocyanine, Indanthrenfarbstoffe, Azofarbstoffe, optische Aufheller und Fluoreszenzfarbstoffe, sowie Pigmente, wie Ruß oder Titandioxid, wobei optische Aufheller und Ruß bevorzugt und optische Aufheller besonders bevorzugt sind.

Falls in den erfindungsgemäßen Zubereitungen Farbmittel (g) enthalten sind, handelt es sich um Mengen von vorzugsweise 0,0001 bis 20 Gewichtsteilen, besonders bevorzugt 0,001 bis 5 Gewichtsteilen, insbesondere 0,01 bis 1 Gewichtsteilen, jeweils bezogen auf 100 Gewichtsteile des Gesamtgewichts der Komponenten (a), (b) und (c). Die erfindungsgemäßen Zubereitungen enthalten bevorzugt keine Farbmittel (g).

Beispiele für gegebenenfalls eingesetzte Polymere (h) sind von Organyloxygruppen freie Polysiloxane, Polyether, Polyurethane oder Polyharnstoffe mit bevorzugt 15 bis 1000 Wiederholungseinheiten, bevorzugt von Organyloxygruppen freie Polysiloxane oder Polyether, besonders bevorzugt von Organyloxygruppen freie Polysiloxane.

Falls Polymere (h) in den erfindungsgemäßen Zubereitungen enthalten sind, handelt es sich um Mengen von vorzugsweise 0,1 bis 500 Gewichtsteilen, besonders bevorzugt 1 bis 100 Gewichtsteilen, insbesondere 5 bis 50 Gewichtsteilen, jeweils bezogen auf 100 Gewichtsteile des Gesamtgewichts der Komponenten (a), (b) und (c). Die erfindungsgemäßen Zubereitungen enthalten bevorzugt keine Polymere (h).

Bei den in den erfindungsgemäßen Zubereitungen enthaltenen Komponenten kann es sich jeweils um eine Art einer solchen Komponente wie auch um ein Gemisch aus mindestens zwei Arten einer jeweiligen Komponente handeln.

Die erfindungsgemäßen Zubereitungen können nach beliebigen und bekannten Verfahren, wie durch einfaches Vermischen der einzelnen Bestandteile, hergestellt werden. Vorzugsweise wird die nach dem erfindungsgemäßen Verfahren erhaltene Reaktionsmischung, die im Wesentlichen aus (a) bicyclischem Guanidin und (b) Silan der allgemeinen Formel (IV) und/oder dessen Teilhydrolysaten und (d) Reaktionsnebenprodukten besteht, mit Alkohol (c) sowie gegebenenfalls den Komponenten (e) bis (h) vermischt. Bevorzugt wird dieses Vermischen bei einer Temperatur von 10 bis 100°C und dem Druck der umgebenden Atmosphäre, also etwa 900 bis 1100 hPa, durchgeführt.

Ein weiterer Gegenstand der vorliegenden Erfindung ist ein Verfahren zur Herstellung der erfindungsgemäßen Zubereitungen durch Mischen der einzelnen Komponenten in beliebiger Reihenfolge.

Die erfindungsgemäßen Zubereitungen sind bei 20°C und 1013 hPa bevorzugt nahezu farblose bis schwach gelbliche und homogene Flüssigkeiten.

Die erfindungsgemäß hergestellten bicyclischen Guanidine sowie die erfindungsgemäßen Zubereitungen können überall da eingesetzt werden, wo auch bisher bicyclische Guanidine eingesetzt wurden, insbesondere als flüssige Katalysatorzubereitung bei der Umsetzung von Hydroxysiloxanen mit Alkoxysilanen (sog. Endcapping), bei der ringöffnenden Polymerisation von Lactonen, Lactamen und cyclischen Carbonaten, zur Überführung von Estern in Amide und von Carbonaten in Harnstoffe, zur Aldolkondensation, und zur Transalkoxylierung von Alkoxysilanen und Alkoxysiloxanen.

Das erfindungsgemäße Verfahren hat den Vorteil, dass bei Einsatz von Alkoxysilanen als Reaktionsmedium und gleichzeitig als Mittel zur Entfernung von Wasser bicyclische Guanidine in hohen Ausbeuten hergestellt werden können.

Ein weiterer Vorteil des erfindungsgemäßen Verfahrens ist, dass auf den Einsatz der chemisch und toxikologisch problematischen Glycolether als Solventien komplett verzichtet werden kann.

Das Verfahren liefert überraschenderweise - im Gegensatz zu Verfahren des Standes der Technik, mit welchen dunkel gefärbte Reaktionsgemische erhalten werden - blassgelbe Reaktionsmischungen.

Das erfindungsgemäße Verfahren hat ferner den Vorteil, dass die eingesetzten Alkoxysilane überraschend gut in der Lage sind, den durch die Umsetzung mit Wasser gebildeten Alkohol aus dem Reaktionsgemisch herauszuschleppen und damit den Prozess der Bildung cyclischer Guanidine zu begünstigen.

Überraschend ist zudem, dass das Produktgemisch, welches cyclische Guanidine und Alkoxysilan bzw. die durch die Reaktion mit Wasser gebildeten Kondensationsprodukte des Alkoxysilans enthält, durch Zusatz von verhältnismäßig geringen Mengen Alkohol verflüssigt werden kann und die erhaltene Zubereitung daher ohne weitere Aufarbeitung des Reaktionsgemisches direkt für katalytische Prozesse eingesetzt werden kann.

Die erfindungsgemäßen Zubereitungen haben den Vorteil, dass sie flüssig sind und sich ausgezeichnet für katalytische Zwecke eignen. Insbesondere besteht durch den Siloxananteil in den erfindungsgemäßen Formulierungen eine sehr gute Mischbarkeit mit Siloxanen, wodurch katalytische Anwendungen in diesem Bereich besonders vorteilhaft sind.

Ein weiterer wirtschaftlicher Vorteil ist, dass die erfindungsgemäße Herstellung von bicyclischen Guanidinen und die Weiterverarbeitung zu der erfindungsgemäßen flüssigen Formulierung ohne Feststoffhandling erfolgt und außerdem keine Abfallprodukte abgetrennt werden müssen.

In den nachfolgenden Beispielen beziehen sich alle Angaben von Teilen und Prozentsätzen, soweit nicht anders angegeben, auf das Gewicht. Sofern nicht anders angegeben, werden die folgenden Beispiele bei einem Druck der umgebenden Atmosphäre, also bei etwa 1000 hPa, und bei Raumtemperatur, also etwa 20°C, bzw. einer Temperatur, die sich beim Zusammengeben der Reaktanden bei Raumtemperatur ohne zusätzliche Heizung oder Kühlung einstellt, durchgeführt. Alle in den Beispielen angeführten Viskositätsangaben sollen sich auf eine Temperatur von 25°C beziehen. Bei den Versuchen wird jeweils mit Stickstoff inertisiert.

### Beispiel 1

409 g (3,11 mol) Bis-(3-aminopropyl)amin und 11 g einer TBD-Lösung in Ethanol mit einem TDB-Gehalt von ca. 19 % wurden bei Raumtemperatur vermischt und unter Rühren innerhalb einer Stunde mit 293 g (3,24 mol) Dimethylcarbonat versetzt. Hierbei wurde ein Temperaturanstieg bis auf 90°C Sumpftemperatur beobachtet. Nach Abklingen der exothermen Reaktion wurde noch 3 Stunden auf 90°C erhitzt, und das gebildete Methanol sowie vorhandenes Ethanol wurde über eine Brücke bei einer Sumpftemperatur bis 139°C abdestilliert.

Der so erhaltene Rückstand wurde mit 1258 g (4,55 mol) i-Octyltriethoxysilan (=2,4,4-Trimethylpentyltriethoxysilan) versetzt, das Gemisch auf 240°C erhitzt und gebildetes Ethanol über eine Brücke abdestilliert. Die Reaktionszeit betrug 12 Stunden. Beim Abkühlen kristallisierte TBD aus dem Sumpf aus. Durch Zugabe von 440 g Ethanol erhielt man eine nahezu farblose homogene TBD-Lösung (Gesamtgewicht 1880 g) mit einem TBD-Gehalt von 19 Gew.-% (387 g TBD, HPLC-Analyse), dies entspricht einer Ausbeute an TBD von 90%.

### Beispiel 2

203 g (1,55 mol) Bis-(3-aminopropyl)amin wurden auf 60°C erwärmt und in einer Stunde unter Rühren mit 146 g (1,62 mol) Dimethylcarbonat versetzt. Hierbei wurde ein Temperaturanstieg bis auf 86°C Sumpftemperatur beobachtet. Es wurden noch 3 Stunden auf 90°C erhitzt und anschließend gebildetes Methanol abdestilliert.

Der so erhaltene Rückstand wurde mit 624 g (2,26 mol) Isooctyltriethoxysilan versetzt, das Gemisch unter Rühren auf 250°C erhitzt und gebildetes Ethanol über eine Brücke abdestilliert. Die Reaktionszeit bei 250°C betrug 9 Stunden. Beim Abkühlen kristallisiert TBD aus dem Sumpf aus. Durch Zugabe von 210 g Ethanol erhielt man eine nahezu farblose homogene TBD-Lösung (Gesamtgewicht 940 g) mit einem TBD-Gehalt von 20 Gew.-% (188 g TBD, HPLC-Analyse), dies entspricht einer Ausbeute an TBD von 87 %.

### Vergleichsbeispiel 3

77,3 g (0,59 mol) Bis-(3-aminopropyl)amin wurden bei Raumtemperatur in einer Stunde unter Rühren mit 55,6 g (0,62 mol) Dimethylcarbonat versetzt. Hierbei wurde ein Temperaturanstieg bis auf 50°C Sumpftemperatur beobachtet. Es wurden noch 3 Stunden auf 90°C erhitzt und anschließend gebildetes Methanol abdestilliert. Man erhielt 109 g Rückstand, welcher mit 232 g eines Gemisches bestehend aus 25 % Tetraethoxysilan, 35 % Hexaethoxydisiloxan, 26 % Octaethoxytrisiloxan, 10 % Octaethoxycyclotetrasiloxan und 4 % Decaethoxytetrasiloxan versetzt und 10 Stunden unter Rühren auf Temperaturen zwischen 210°C und 235°C erhitzt und dabei Ethanol abdestilliert wurde. Nach dem Erkalten wurde das das kristalline TBD enthaltende dreiphasige (zwei flüssige Phasen und Feststoff) dunkel gefärbte Reaktionsgemisch mit 60 g Ethanol versetzt. Die untere zähflüssige Phase blieb zunächst bestehen und löste sich nach Erwärmen auf 70°C / 10 Stunden auf. Die erhaltene homogene Lösung (Gesamtgewicht 275 g) hatte einen TBD-Gehalt (HPLC) von 10,4 %, entsprechend 28,6 g TBD (35 %).

### Beispiel 4

109 g des Produktes aus der Umsetzung von Bis-(3-aminopropyl)-amin mit Dimethylcarbonat nach Beispiel 2 wurden unter Rühren mit 251 g eines Alkoxysilangemisches bestehend aus 30 % Diet-hoxy-di-(2-butoxy)silan, 50 % Ethoxy-tri-(2-butoxy)silan und 20 % Tetra-(2-butoxy)silan versetzt und 10 Stunden unter Rühren auf Temperaturen zwischen 210°C und 235°C erhitzt und dabei Ethanol und 2-Butanol abdestilliert. Nach dem Erkalten wurde das kristalline TBD enthaltende Reaktionsgemisch mit 43 g Ethanol versetzt. Es bildete sich eine homogene Lösung (Gesamtgewicht 386 g), der TBD-Gehalt (HPLC) betrug 22,4 %, entspricht 86,5 g TBD (90 %).

### Beispiel 5

30,0 g (0,29 mol) Diethylentriamin wurden auf 50°C erwärmt und innerhalb von 45 Minuten unter Rühren mit 21,8 g (0,24 mol) Dimethylcarbonat versetzt. Hierbei wurde ein Temperaturanstieg bis auf 50°C Sumpftemperatur beobachtet. Es wurde noch 3 Stunden auf 90°C erhitzt und anschließend gebildetes Methanol bei Sumpftemperatur zwischen 125-145°C abdestilliert. Man erhielt 40,2 g Rückstand, welcher mit 200 g i-Octyltriethoxysilan versetzt und unter Rühren über 20 Std auf 220°C bis 250°C erhitzt wurde. Hierbei wird das gebildete Ethanol abdestilliert. Nach dem Erkalten des Reaktionsansatzes wird dieser mit 38 g Ethanol versetzt. Es ergab sich eine klare homogene Lösung mit einem TBO-Gehalt von 6,1 % (HPLC).

### Beispiel 6

117 g des Produktes aus der Umsetzung von Bis-(3-aminopropyl)-amin mit Dimethylcarbonat nach Beispiel 2 wurden mit 300 g i-Octyltriethoxysilan versetzt, das Gemisch unter Rühren auf Temperaturen zwischen 230 und 250°C erhitzt und gebildetes Ethanol (49 g) über eine Brücke abdestilliert. Die Reaktionszeit betrug 13 Stunden. Anschließend wurde auf ca. 190°C abgekühlt und aus der bei dieser Temperatur homogenen Reaktionsmischung eine Probe über HPLC untersucht. Der Gehalt an TBD betrug 23 %. Dies entspricht einer TBD Menge von 85 g (99 %). Beim Abkühlen auf Umgebungstemperatur kristallisierte TBD aus dem Sumpf aus. Durch Zugabe von 71 g Ethanol erhielt man eine nahezu farblose homogene TBD-Lösung.

### Beispiel 7

204 g (1,55 mol) Bis-(3-aminopropyl)amin wurden mit 14,4 g der nach Beispiel 1 erhaltenen TBD-Lösung versetzt, auf 90°C erwärmt und innerhalb einer Stunde 191 g (1,62 mol) Diethylcarbonat unter Rühren zugegeben. Hierbei wurde ein Temperaturanstieg bis auf 102°C Sumpftemperatur beobachtet. Es wurde noch 3 Stunden auf 105°C erhitzt (Rückfluss Ethanol) und anschließend gebildetes Ethanol (142 g) bei Normaldruck und Sumpftemperatur von 110 bis 160°C abdestilliert. Man erhielt 271 g Rückstand (Gehalt an cyclischem Harnstoff 90 %). Hiervon wurden 117 g (0,67 mmol) mit 248 g i-Octyltriethoxysilan versetzt, das Gemisch unter Rühren auf 245°C erhitzt und gebildetes Ethanol über eine Brücke abdestilliert. Die Reaktionszeit bei 245°C betrug 12 Stunden. Beim Abkühlen auf Umgebungstemperatur kristallisierte TBD aus dem Sumpf aus. Durch Zugabe von 87 g Ethanol erhielt man eine homogene TBD-Lösung (Gesamtgewicht 367 g) mit einem TBD-Gehalt von 18 Gew. % (66 g TBD, HPLC-Analyse), dies entspricht einer Ausbeute an TBD von 72 %.

### Beispiel 8

103 g (0,78 mol) Bis-(3-aminopropyl)amin wurden mit 7,2 g der nach Beispiel 1 erhaltenen TBD-Lösung und mit 100 g i-Octyltriethoxysilan versetzt, auf 90°C erwärmt und innerhalb einer Stunde unter Rühren 95,5 g (0,80 mol) Diethylcarbonat zugegeben. Hierbei wurde ein Temperaturanstieg bis auf 106°C Sumpftemperatur beobachtet. Es wurde noch 3 Stunden auf 105°C erhitzt (Rückfluss Ethanol) und anschließend gebildetes Ethanol (73 g) bei Normaldruck und Sumpftemperatur von 110 bis 160°C abdestilliert. Man erhielt 233 g eines zweiphasigen Rückstands. Dieser wurde mit 182 g *i*-Octyltriethoxysilan versetzt, das Gemisch unter Rühren auf 245°C erhitzt und gebildetes Ethanol (ca. 70 g) über eine Brücke abdestilliert. Die Reaktionszeit bei 245°C betrug 12 Stunden. Beim Abkühlen auf Umgebungstemperatur kristallisierte TBD aus dem Sumpf aus. Durch Zugabe von 100 g Ethanol erhielt man eine homogene TBD-Lösung (Gesamtgewicht 445 g) mit einem TBD-Gehalt von 22 Gew. % (98 g TBD, HPLC-Analyse), dies entspricht einer Ausbeute an TBD von 91 %.

## Patentansprüche

1. Verfahren zur Herstellung bicyclischer Guanidine durch Umsetzung von (A) Dialkylentriamin mit (B) Dialkylcarbonat in Gegenwart von (C) Silan der Formel
Si(OR^{x})ₒR^{y}₍₄₋ₒ₎ (IV)
und/oder dessen Teilhydrolysate, wobei
R^{x} gleich oder verschieden sein können und einwertige, gegebenenfalls substituierte Kohlenwasserstoffreste mit 2 bis 10 Kohlenstoffatomen darstellen,
R^{y} gleich oder verschieden sein können und einwertige, gegebenenfalls substituierte Kohlenwasserstoffreste mit 1 bis 30 Kohlenstoffatomen, bei denen einzelne nicht an Silicium gebundene CH₂-Gruppierungen durch Sauerstoff ersetzt oder durch Silylgruppen substituiert sein können, darstellen und
o 1, 2, 3 oder 4 ist,
mit der Maßgabe, dass bei Silanen der Formel (IV) mit o=4 mindestens zwei Reste R^{x} die Bedeutung von einwertigem, gegebenenfalls substituiertem Kohlenwasserstoffrest mit 3 bis 10 Kohlenstoffatomen haben.

2. Verfahren nach Anspruch 1, **dadurch gekennzeichnet, dass** es sich bei den Dialkylentriaminen (A) um solche der allgemeinen Formel
H₂N-(CR^{a}₂)ₘ-CR^{a}₂-NH-CR^{a}₂-(CR^{a}₂)ₙ-NH₂ (II)
handelt, wobei
m und n unabhängig voneinander 1, 2, 3 oder 4 sind und
R^{a} gleich oder verschieden sein kann und Wasserstoffatom oder einwertige Kohlenwasserstoffreste darstellt, wobei einzelne Methylengruppen durch Sauerstoff oder durch Gruppierungen -NH- oder -NR^{d}- ersetzt sein können, wobei R^{d} einwertige, gegebenenfalls substituierte Kohlenwasserstoffreste mit 2 bis 10 Kohlenstoffatomen bedeutet.

3. Verfahren nach Anspruch 1 oder 2, **dadurch gekennzeichnet, dass** es sich bei den Dialkylcarbonaten (B) um solche der allgemeinen Formel
R^{b}O-CO-OR^{b} (III)
handelt, wobei
R^{b} gleich oder verschieden sein kann und ein- oder zweiwertige, aliphatisch gesättigte Kohlenwasserstoffreste darstellt.

4. Verfahren nach einem oder mehreren der Ansprüche 1 bis 3, **dadurch gekennzeichnet, dass** Komponente (C) in Mengen von 30 bis 1000 Gewichtsteilen, bezogen auf 100 Gewichtsteile Dialkylentriamin (A), eingesetzt wird.

5. Verfahren nach einem oder mehreren der Ansprüche 1 bis 4, **dadurch gekennzeichnet, dass** in einer ersten Stufe die Komponenten (A) und (B) sowie gegebenenfalls Komponenten (D), (E), (f), (g) und (h) vermischt und reagieren gelassen werden und dann in einer zweiten Stufe Komponente (C) sowie gegebenenfalls Komponenten (D), (E), (f), (g) und (h) zugegeben und auf eine Temperatur größer 200°C erhitzt werden.

6. Zubereitung bestehend aus
(a) bicyclischem Guanidin,
(b) Silan der allgemeinen Formel (IV) und/oder dessen Teilhydrolysate,
(c) Verbindung R^{c}OH, wobei Rest R^{c} gleich Wasserstoffatom oder einwertige, gegebenenfalls substituierte Kohlenwasserstoffreste mit 1 bis 30 Kohlenstoffatomen, die mit Sauerstoff unterbrochen sein können, darstellt,
gegebenenfalls (d) Reaktionsnebenprodukte,
gegebenenfalls (e) organische, von an aliphatische Kohlenstoffatome gebundenen Hydroxylgruppen freie Lösungsmittel,
gegebenenfalls (f) Füllstoffe,
gegebenenfalls (g) Farbmittel und
gegebenenfalls (h) Polymere.

7. Zubereitung gemäß Anspruch 6, **dadurch gekennzeichnet, dass** der Gehalt an bicyclischen Guanidinen (a) 2 bis 35 Gew.-% beträgt.

8. Zubereitung gemäß Anspruch 6 oder 7, **dadurch gekennzeichnet, dass** der Gehalt an Silanen und/oder Siloxanen (b) 20 bis 90 Gew.-%, beträgt.

9. Zubereitung gemäß einem oder mehreren der Ansprüche 6 bis 8, **dadurch gekennzeichnet, dass** der Gewichtsanteil der Komponenten (a), (b), (c) und gegebenenfalls (d) in den erfindungsgemäßen Zusammensetzungen mindestens 80 Gew.-% beträgt.

10. Verfahren zur Herstellung der Zubereitungen gemäß einem oder mehreren der Ansprüche 6 bis 9 durch Mischen der einzelnen Komponenten in beliebiger Reihenfolge.

## Claims

1. Process for producing bicyclic guanidines by reacting (A) dialkylenetriamine with (B) dialkyl carbonate in the presence of (C) silane of the formula
Si (OR^{x})ₒR^{y}₍₄₋ₒ₎ (IV)
and/or partial hydrolysates thereof,
where
R^{x} may be identical or different and are monovalent, optionally substituted hydrocarbon radicals having 2 to 10 carbon atoms,
R^{y} may be identical or different and are monovalent, optionally substituted hydrocarbon radicals having 1 to 30 carbon atoms in which individual CH₂ moieties not bonded to silicon may be replaced by oxygen or substituted by silyl groups and
o is 1, 2, 3 or 4,
with the proviso that, in the case of silanes of the formula (IV) where o = 4, at least two radicals R^{x} represent a monovalent, optionally substituted hydrocarbon radical having 3 to 10 carbon atoms.

2. Process according to Claim 1, **characterized in that** the dialkylenetriamines (A) are of the general formula H₂N- (CR^{a}₂)ₘ-CR^{a}₂-NH-CR^{a}₂- (CR^{a}₂)ₙ-NH₂ (II) where
m and n are independently 1, 2, 3 or 4 and
R^{a} may be identical or different and is hydrogen atom or monovalent hydrocarbon radicals in which individual methylene groups may be replaced by oxygen or by -NH- or -NR^{d} - moieties, where R^{d} represents monovalent, optionally substituted hydrocarbon radicals having 2 to 10 carbon atoms.

3. Process according to Claim 1 or 2, **characterized in that** the dialkyl carbonates(B) are of the general formula
R^{b}O-CO-OR^{b} (III)
where
R^{b} may be identical or different and is mono- or divalent, aliphatically saturated hydrocarbon radicals.

4. Process according to one or more of Claims 1 to 3, **characterized in that** component(C) is used in amounts of 30 to 1000 parts by weight based on 100 parts by weight of dialkylenetriamine (A).

5. Process according to one or more of Claims 1 to 4, **characterized in that** components (A) and (B) and optionally components (D), (E), (f), (g), and (h) are mixed and allowed to react in a first step, which is followed by a second step in which component (C) and optionally components (D), (E), (f), (g) and (h) are added and the mixture is heated to a temperature greater than 200°C.

6. Preparation consisting of
(a) bicyclic guanidine,
(b) silane of the general formula (IV) and/or the partial hydrolysates thereof,
(c) compound R^{c}OH, wherein R^{c} is hydrogen atom or monovalent, optionally substituted hydrocarbon radicals having 1 to 30 carbon atoms, which may be interrupted by oxygen,
optionally (d) reaction side products,
optionally (e) organic solvents free of hydroxyl groups attached to aliphatic carbon atoms,
optionally (f) fillers,
optionally (g) colorants, and
optionally (h) polymers.

7. Preparation according to Claim 6, **characterized in that** the content of bicyclic guanidines (a) is 2% to 35% by weight.

8. Preparation according to Claim 6 or 7, **characterized in that** the content of silanes and/or siloxanes (b) is 20% to 90% by weight.

9. Preparation according to one or more of Claims 6 to 8, **characterized in that** the proportion by weight of components (a), (b), (c), and optionally (d) in the compositions according to the invention is at least 80% by weight.

10. Process for producing the preparations according to one or more of Claims 6 to 9 by mixing the individual components in any desired order.

## Revendications

1. Procédé de préparation de guanidines bicycliques par réaction de (A) dialkylènetriamine avec (B) un carbonate de dialkyle
en présence de (C) un silane de Formule
si (OR^{x})ₒR^{y}₍₄₋ₒ₎ (IV)
et/ou ses hydrolysats partiels,
dans lequel
les radicaux R^{x} peuvent être identiques ou différents et représenter des radicaux hydrocarbonés monovalents, éventuellement substitués, ayant 2 à 10 atomes de carbone,
les radicaux R^{y} peuvent être identiques ou différents et représentent des radicaux hydrocarbonés monovalents, éventuellement substitués, ayant 1 à 30 atomes de carbone, dans lesquels des groupements CH₂ individuels non liés au silicium peuvent être remplacés par un oxygène ou substitués par des groupes silyle, et
o représente 1, 2, 3 ou 4,
à la condition que, dans le cas des silanes de Formule (IV) avec o = 4, au moins deux radicaux R^{x} aient la signification d'un radical hydrocarboné monovalent, éventuellement substitué, ayant 3 à 10 atomes de carbone.

2. Procédé selon la revendication 1, **caractérisé en ce que**, pour ce qui concerne les dialkylènetriamines (A), il s'agit de celles de Formule générale
H₂N-(CR^{a}₂)ₘ-CR^{a}₂-NH-CR^{a}₂-(CR^{a}₂)ₙ-NH₂ (II)
dans laquelle
m et n représentent indépendamment l'un de l'autre 1, 2, 3 ou 4 et
les radicaux R^{a} peuvent être identiques ou différents et représentent atome d'hydrogène ou des radicaux hydrocarbonés monovalents, des groupes éthylène individuels pouvant être remplacés par un oxygène ou par des groupements -NH- ou -NR^{d}-, R^{d} représentant des radicaux hydrocarbonés monovalents, éventuellement substitués, ayant 2 à 10 atomes de carbone.

3. Procédé selon la revendication 1 ou 2, **caractérisé en ce que**, pour ce qui concerne les carbonates de dialkyle (B), il s'agit de ceux de Formule générale
R^{b}O-CO-OR^{b} (III)
dans laquelle
les radicaux R^{b} peuvent être identiques ou différents et représentent des radicaux hydrocarbonés mono- ou divalents, à saturation aliphatique.

4. Procédé selon l'une ou plusieurs des revendications 1 à 3, **caractérisé en ce que** le composant (C) est utilisé en des quantités de 30 à 1 000 parties en poids, pour 100 parties en poids de la dialkylènetriamine (A).

5. Procédé selon l'une ou plusieurs des revendications 1 à 4, **caractérisé en ce que**, dans une première étape, on mélange les composants (A) et (B), ainsi qu'éventuellement les composés (D), (E), (f), (g) et (h) et on les fait réagir, puis, dans une deuxième étape, on ajoute le composant (C) ainsi qu'éventuellement les composants (D), (E), (f), (g) et (h), et on chauffe à une température supérieure à 200 °C.

6. Préparation consistant en
(a) une guanidine bicyclique,
(b) un silane de Formule générale (IV) et/ou ses hydrolysats partiels,
(c) un composé R^{c}OH, dans lequel R^{c} représente atome d'hydrogène ou des radicaux hydrocarbonés monovalents, éventuellement substitués, ayant 1 à 30 atomes de carbone, qui peuvent être interrompus par un oxygène,
éventuellement (d) des sous-produits de réaction,
éventuellement (e) des solvants organiques, exempts de groupes hydroxyle liés à des atomes de carbone aliphatiques,
éventuellement (f) des charges,
éventuellement (g) des colorants et
éventuellement (h) des polymères.

7. Préparation selon la revendication 6, **caractérisée en ce que** la teneur en guanidines bicycliques (a) est de 2 à 35 % en poids.

8. Préparation selon la revendication 6 ou 7, **caractérisée en ce que** la teneur en silanes et/ou en siloxanes (b) est de 20 à 90 % en poids.

9. Préparation selon l'une ou plusieurs des revendications 6 à 8, **caractérisée en ce que** la proportion pondérale des composants (a), (b), (c) et éventuellement (d) dans les compositions selon l'invention est d'au moins 80 % en poids.

10. Procédé de fabrication des préparations selon l'une ou plusieurs des revendications 6 à 9 par mélange des composants individuels dans un ordre quelconque.
